# EUROPEAN PATENT SPECIFICATION

(11) **EP 0 575 515 B1**
(45) Date of publication and mention of the grant of the patent: **21.07.1999**
(21) Application number: 92908653.6
(22) Date of filing: 27.02.1992
(51) Int. Cl.: C12Q 1/00, C12Q 1/26, C12N 9/96, C12Q 1/54, C12Q 1/60

(54) **STABILIZATION OF ENZYME CONTAINING REAGENT COMPOSITION FOR DETERMINATION OF AN ANALYTE**
STABILISIERUNG EINES ENZYM ENTHALTENDEN REAGENZ ZUR BESTIMMUNG EINES ANALYTEN
STABILISATION D'UN REACTIF CONTENANT UNE ENZYME POUR LA DETERMINATION D'UN ANALYTE

(30) Priority: 27.02.1991 US 661716
(43) Date of publication of application: 29.12.1993
(73) Proprietor: BOEHRINGER MANNHEIM CORPORATION, Indianapolis, Indiana 46250-0528 (US)
(72) Inventor: KOST, Kent, M., Fishers, IN 46038 (US)
(74) Representative: Jung, Michael, Dr.
(86) International application number: US9201662
(87) International publication number: WO9215701

(56) References cited:
- WO-A-91/09139
- WO-A-92/15704
- US-A- 4 576 913
- US-A- 4 929 545

## Description

### FIELD OF THE INVENTION

This invention relates generally to a method for improving the stability of reagent compositions useful in determining an analyte in a sample.

### BACKGROUND AND PRIOR ART

The central concern of clinical chemistry is the qualitative and quantitative determination of specific analytes in samples. Of special concern is the analysis of body fluid samples, such as blood, serum, urine and so forth. Determination of the presence and/or amount of various analytes, followed by comparison to established parameters determines diagnosis of diseased or abnormal states.

The literature on analytical determination of body fluid samples is large, as the art has investigated the determination of, e.g., glucose, cholesterol, creatine, sarcosine, urea and other substances in samples of blood, serum, urine and so forth.

There are many types of assays for making such analytical determinations. One type of assay is based on a sequence of reactions. The first reaction is between an analyte and an enzyme which is specific for that analyte. In that reaction the enzyme "oxidizes" the analyte, causing it to lose an electron. In a subsequent reaction the electron is accepted by a mediator which is thus reduced. The reduced mediator can then be used in a variety of indicator systems to determine the presence and concentration of the analyte.

One such indicator system is described in commonly asigned US Patent No. 4,929,545. In that system a ferricyanide is reduced to a ferrocyanide by the electron lost by the analyte. The ferrocyanide combines with a ferric compound to form a color. The presence and degree of color change are indications of the presence and concentration, respectively, of the analyte in a specimen.

The degree of color change can be measured by a reflectance photometer. The photometer is normally a part of a meter which uses an internal "look-up" table or an algorhythm to convert the change in reflectance to analyte concentration.

Other similar systems used ferrocene or one of its analogs as a mediator.

Another system for using the reduced mediator to determine the presence or concentration of an analyte is described in commonly assigned copending U.S. patent application USSN 07/451,671, filed December 15, 1989, published as WO 91/09139. In that system the reduced mediator is re-oxidized by the application of a current, and the decay in the current after a specific time is measured and used to determine the concentration of the analyte.

In either system increased stability of the reagent containing the mediator is always desirable. Stability is necessary to obtain a commercially interesting shelf life. In the system described in US Patent No. 4,929,545, as in any system where change in light reflectance is measured over a range, it is desirable to increase the range. A larger range increases the size of the increments of measured reflectance and, thus, increases accuracy.

### SUMMARY OF THE INVENTION

It is an object of the present invention to increase the stability of of a reagent containing a reducible mediator.

This and other objects are accomplished by a reagent composition which comprises a reducible mediator and an alkali metal dichromate as oxidising agent which will improve the stability of the reagent prior to use in an assay and which will not significantly interfere with the reduction of the mediator during performance of the assay.

This invention is based on the surprising and unexpected discoveries that such an oxidizing agent can be selected and that the selected oxidizing agent increases the range of change in reflectance of an ingredient which changes color in response to reduction of the mediator.

Any alkali metal dichromate which will inhibit premature reduction of the reducible mediator but which will not compete successfully with the reduction of the mediator during performance of an assay is useful in the invention. In the color-based assay described in US Patent No. 4,929,545, when the mediator is a ferricyanide, the indicator is a soluble ferric compound such as ferric chloride or ferric sulphate and the enzyme is glucose oxidase or cholesterol oxidase, it has been discovered that 1.3 x 10⁻⁴ g per kU of enzyme to 17.5 x 10⁻⁴ g per kU of enzyme of an alkali metal dichromate, such as sodium or potassium dichromate is suitable for use in the invention.

Thus, a preferred reagent for use in such color-based assays for glucose in blood includes potassium ferricyanide, a soluble ferric compound, glucose oxidase and potassium dichromate in a concentration of from a 1.3 x 10⁻⁴ to about 17.5 x 10⁻⁴ grams per kU of glucose oxidase. Such a reagent will have a stability many times greater than the same reagent without the dichromate and will have an increased dynamic range of change in the reflectance region of interest.

The operation of the oxidizing agent with regard to improved stability is not completely understood, although it is believe that the oxidizing agent oxidizes impurities in the other reagent components which might cause premature color change in the indicator. It is also believed that the oxidizing agent stabilizes the mediator with regard to the reducing effects of light, heat and humidity but not with regard to the reducing effects of the electron provided by the reaction between the analyte and the enzyme, surprisingly all without inactivating the enzyme.

The operation of the oxidizing agent to increase the dynamic range of the reagent dose response curve is not fully understood at the present time. Although it is an observed effect, it would be expected that, in the preferred embodiment for example, the oxidizing agent would limit the range of color change because it would act on the reduced ferrocyanide to suppress the reaction between the ferrocyanide and the ferric compound to produce Prussian blue.

It has been found through experimentation that trace quantities of a dichromate will improve the stability of the reagent, that quantities of 1.3 x 10⁻⁴ gram per kU of enzyme will increase stability about twelve-fold at elevated temperatures, and that amounts up to 17.5 x 10⁻⁴ grams per kU of enzyme can be used without hindering color development.

It has also been found that quantities of dichromate as small as 0.9 x 10⁻⁴ grams per kU of enzyme will increase the dynamic range of the reflectance region of interest by about five percentage points, which is an increase of about 10% in the size of the range.

The reagent composition of the present invention has useful commercial application in assays for components of fluid samples. A preferred use is in an assay device such as is described in commonly assigned patent application filed on even date herewith as USSN 07/661,788 by Ralph McCroskey et al., now US Patent No. 5,271,895 where the reagent is coated on a support in a novel teststrip architecture. In that embodiment the reagent layer is contacted by whole blood which is held against the reagent layer in a predetermined volume.

### DETAILED DESCRIPTION AND EXAMPLES

The preferred embodiments of the invention are illustrated in the following examples which are intended to be illuminative, but not limiting.

### EXAMPLE 1

Test strips were prepared and used in order to show the use of the invention in reflectance assays.

A coating mass was prepared which contained, per kilogram of the coating mass, 30 g. 4-amino butyric acid, 3.9 g. of ferric sulfate, 36 g. of ferricyanide salt and 1,000 kU of glucose oxidase. Nonreactive ingredients which made up the remainder of the mass included TiO₂ as a white pigment, TWEEN®-20 (nonionic surfactant), PROPIOFAN®-70D (film former) and NATROSOL® (thickening agent). The pH of this mass was 4.1. of the coating mass in concentrations ranging from a trace to 17.52 x 10⁻⁴ grams as shown in Tables 1a and 1b, below. Some of the aliquots were then subjected to accelerated degradation testing in a thermal stress chamber. The accelerated testing subjects the reagent to elevated temperatures in order to predict a normal shelf life.

After stressing, the aliquots were examined for stability. Color change in the reagent before contact with a fluid sample containing an analyte specific for the enzyme indicates instability and diminished shelf life. Concentrations of dichromate in the various aliquots, the duration of the test, the elevated temperature of the stress chamber and the resulting increase in shelf life over the aliquoit with no dichromate is shown in Table 1a, below. The observations indicate that the addition of a dichromate in even trace amounts can improve significantly, while greater amounts will improve stability for longer times.

Other aliquots were subjected to samples of a glucose-containing control solution which contained glucose in the same range as normal human blood. Table 1b shows the dichromate concentrations of the various aliquots and indicates whether color development is acceptable or hindered. The results indicate that the dichromate does not hinder color development of the reagent until relatively high concentrations are reached.

**TABLE 1A**

| Dichromate Concentration (grams/kU of enzyme) | Stability (normalized) |
|---|---|
| 0 | x @ 25°C. y @ 35° C. |
| | |
| trace | 3x |
| | 3y |
| | |
| 0.4 x 10⁻⁴ | 5x |
| | 8y |
| | |
| 1.3 x 10⁻⁴ | 6x |
| | 12y |

**TABLE 1B**

| Dichromate grams/ kU enzume | Color Performance |
|---|---|
| 4.83 x 10⁻⁴ | Acceptable |
| 8.76 x 10⁻⁴ | Acceptable |
| 13.14 x 10⁻⁴ | Acceptable |
| 17.14 x 10⁻⁴ | Color Development Slowed. |

This experiment shows that the addition of a soluble dichromate to a reagent containing a mediator, an enzyme specific for an analyte and an indicator which reacts with the reduced form of the mediator to produce a color will improve the stability of the reagent without inactivating the enzyme and without supressing color development over a useful range of concentrations.

### EXAMPLE 2

Teststrips were prepared and used in order to show the improvement in the range of change in reflectance when the dichromate oxidizer is added to the reagent.

Two batches of test strip reagent is prepared as in Example 1. One batch contains no dichromate, and the other batch contains about 0.9 x 10⁻⁴ grams of dichromate per kU of enzyme. Each batch is devided into aliquots and coated onto a transparent carrier layers which are part of test strips. Test strips made with each batch of reagent are subjected to samples of a glucose solution containing differing concentrations of glucose. The layers were then interrogated from the side opposite the coated side by a reflectance photometer. The ranges of reflectance of light at 660 nM for reagent containing the dichromate and for reagent not containing the dichromate are shown In Table 2, below.

**TABLE 2**

| Reagent | % Reflectance at 0 mg/dl glucose | % Reflectance at 500 mg/dl glucose |
|---|---|---|
| Without dichromate | 53 | 18 |
| With dichromate | 58 | 18 |

This experiment shows that the addition of the dichromate to the reagent increases the dynamic range of change in reflectance by five percentage points, which is an increase of about 10 percent in the size of the range.

The present invention has been disclosed in the above teachings and examples with sufficient clarity and conciseness to enable one skilled in the art to make and use the invention and to know the best mode for carrying out the invention.

## Claims

1. Use of an alkali metal dichromate as oxidizing agent for improving the stability of a reagent composition useful in an assay for the determination of an analyte in a liquid sample, which reagent composition comprises an enzyme which will act on analyte present in the sample and a ferricyanide compound as soluble mediator which will be reduced in response to the action of the enzyme on the analyte, characterized in that the reagent composition further comprises from about 1.3 x 10-4 grams per kU of enzyme to about 17.5 x 10-4 grams per kU of enzyme of an alkali metal dichromate which is soluble in the reagent composition and which will not successfully compete with the reduction of the ferricyanide compound in response to the action of enzyme on analyte that may be present in the sample but which will inhibit the premature reduction of the ferricyanide compound.

2. The use of Claim 1 wherein the dichromate is potassium dichromate.

3. The use of Claim 1 wherein the analyte is glucose and the enzyme is glucose oxidase.

4. The use of Claim 1 wherein the analyte is cholesterol and the enzyme is cholesterol oxidase.

5. The use of Claim 1, wherein the reagent composition further comprises a soluble ferric compound which reacts with a ferrocyanide compound to form a visually or optically detectable reaction product, and a buffer which does not prevent formation of the reaction product, wherein the composition has a pH of from about 3.0 to about 6.0.

6. The use of Claim 5 wherein the enzyme is glucose oxidase or cholseterol oxidase.

7. Method for improving the stability of a composition which comprises an enzyme and a ferricyanide compound as a soluble mediator which will be reduced in response to the action of the enzyme on an analyte for which it is specific, the method comprising adding to the composition from about 1.3 x 10-4 grams per kU of enzyme to about 17.5 x 10-4 grams per kU of enzyme of an alkali metal dichromate which is soluble in the reagent composition and which will not successfully compete with the reduction of the ferricyanide compound in response to the action of enzyme on analyte that may be present in the sample but which will inhibit the premature reduction of the ferricyanide compound.

8. The method of Claim 7 wherein the alkali metal dichromate is selected from the group consisting of sodium dichromate and potassium dichromate.

9. The method of Claim 7 wherein the enzyme is glucose oxidase or cholesterol oxidase.

10. The method of Claim 7 wherein the enzyme is glucose oxidase and the alkali metal dichromate is potassium dichromate.

11. The method of Claim 7, wherein the composition further comprises a soluble ferric compound which reacts with a ferrocyanide compound to form a visually or optically detectable reaction product, and a buffer which does not prevent formation of the reaction product, wherein the compositiion has a pH of from about 3.0 to about 7.0.

12. The method of Claim 11 wherein the alkali metal dichromate is selected from the group consisting of potassium dichromate and sodium dichromate.

13. The method of Claim 11 wherein the enzyme is glucose oxidase or cholesterol oxidase.

14. The method of Claim 13 wherein the enzyme is glucose oxidase and the alkali metal dichromate is potassium dichromate.

## Patentansprüche

1. Verwendung eines Alkalimetalldichromats als Oxidationsmittel zur Verbesserung der Stabilität einer Reagenzienzusammensetzung, die bei einem Assay zur Bestimmung eines Analyten in einer flüssigen Probe brauchbar ist, wobei die Reagenzienzusammensetzung ein Enzym umfaßt, das auf einen in der Probe vorhandenen Analyten einwirkt, sowie eine Cyanoferrat(III)-Verbindung als löslichen Vermittler, die infolge der Einwirkung des Enzyms auf den Analyten reduziert wird, dadurch gekennzeichnet, daß die Reagenzienzusammensetzung des weiteren etwa 1,3·10⁻⁴ g pro kE Enzym bis etwa 17,5·10⁻⁴ g pro kE Enzym eines Alkalimetalldichromats umfaßt, das in der Reagenzienzusammensetzung löslich ist und nicht erfolgreich mit der Reduktion der Cyanoferrat(III)-Verbindung infolge Einwirkung des Enzyms auf den möglicherweise in der Probe vorhandenen Analyten konkurriert, sondern die vorzeitige Reduktion der Cyanoferrat(III)-Verbindung hemmt.

2. Verwendung nach Anspruch 1, wobei das Dichromat Kaliumdichromat ist.

3. Verwendung nach Anspruch 1, wobei der Analyt Glucose ist und das Enzym Glucoseoxidase ist.

4. Verwendung nach Anspruch 1, wobei der Analyt Cholesterin ist und das Enzym Cholesterinoxidase ist.

5. Verwendung nach Anspruch 1, wobei die Reagenzienzusammensetzung des weiteren eine lösliche Eisen(III)-Verbindung umfaßt, die mit einer Cyanoferrat(II)-Verbindung reagiert, um ein visuell oder optisch erfaßbares Reaktionsprodukt zu bilden, sowie einen Puffer, der die Bildung des Reaktionsprodukts nicht verhindert, wobei die Zusammensetzung einen pH von etwa 3,0 bis etwa 6,0 aufweist.

6. Verwendung nach Anspruch 5, wobei das Enzym Glucoseoxidase oder Cholesterinoxidase ist.

7. Verfahren zur Verbesserung der Stabilität einer Zusammensetzung, die ein Enzym und eine Cyanoferrat(III)-Verbindung als löslichen Vermittler umfaßt, die infolge der Einwirkung des Enzyms auf einen für dieses spezifischen Analyten reduziert wird, umfassend das Versetzen der Zusammensetzung mit etwa 1,3·10⁻⁴ g pro kE Enzym bis etwa 17,5·10⁻⁴ g pro kE Enzym eines Alkalimetalldichromats, das in der Reagenzienzusammensetzung löslich ist und nicht erfolgreich mit der Reduktion der Cyanoferrat(III)-Verbindung infolge Einwirkung des Enzyms auf den möglicherweise in der Probe vorhandenen Analyten konkurriert, sondern die vorzeitige Reduktion der Cyanoferrat(III)-Verbindung hemmt.

8. Verfahren nach Anspruch 7, wobei das Alkalimetalldichromat ausgewählt ist aus der Gruppe bestehend aus Natriumdichromat und Kaliumdichromat.

9. Verfahren nach Anspruch 7, wobei das Enzym Glucoseoxidase oder Cholesterinoxidase ist.

10. Verfahren nach Anspruch 7, wobei das Enzym Glucoseoxidase ist und das Alkalimetalldichromat Kaliumdichromat ist.

11. Verfahren nach Anspruch 7, wobei die Zusammensetzung des weiteren eine lösliche Eisen(III)-Verbindung umfaßt, die mit einer Cyanoferrat(II)-Verbindung reagiert, um ein visuell oder optisch erfaßbares Reaktionsprodukt zu bilden, sowie einen Puffer, der die Bildung des Reaktionsprodukts nicht verhindert, wobei die Zusammensetzung einen pH von etwa 3,0 bis etwa 7,0 aufweist.

12. Verfahren nach Anspruch 11, wobei das Alkalimetalldichromat ausgewählt ist aus der Gruppe bestehend aus Kaliumdichromat und Natriumdichromat.

13. Verfahren nach Anspruch 11, wobei das Enzym Glucoseoxidase oder Cholesterinoxidase ist.

14. Verfahren nach Anspruch 13, wobei das Enzym Glucoseoxidase ist und das Alkalimetalldichromat Kaliumdichromat ist.

## Revendications

1. Utilisation d'un dichromate de métal alcalin comme agent d'oxydation pour améliorer la stabilité d'une composition de réactifs, utilisée dans une analyse pour la détermination d'un analyte dans un échantillon liquide, ladite composition de réactifs contenant une enzyme qui agira sur l'analyte présent dans l'échantillon et un composé de ferricyanure comme médiateur soluble qui sera réduit en réponse à l'action de l'enzyme sur l'analyte, caractérisée en ce que la composition de réactifs contient en outre environ 1,3x10⁻⁴ grammes par kU d'enzyme à environ 17,5x10⁻⁴ grammes par kU d'enzyme d'un dichromate de métal alcalin qui est soluble dans la composition de réactifs et qui ne réussira pas à entrer en concurrence avec la réduction du composé de ferricyanure, en réponse à l'action de l'enzyme sur l'analyte qui peut être présent dans l'échantillon, mais qui inhibera la réduction prématurée du composé de ferricyanure.

2. Utilisation de la revendication 1, le dichromate étant du dichromate de potassium.

3. Utilisation de la revendication 1, l'analyte étant du glucose et l'enzyme de la glucose oxydase.

4. Utilisation de la revendication 1, l'analyte étant du cholestérol et l'enzyme de la cholestérol oxydase.

5. Utilisation de la revendication 1, la composition de réactifs contenant en outre un composé ferrique soluble qui réagit avec un composé ferrocyanure, afin de former un produit de réaction visuellement ou optiquement détectable, et un tampon qui n'empêche pas la formation du produit de réaction, la composition présentant un pH compris entre environ 3,0 et environ 6,0.

6. Utilisation de la revendication 5, l'enzyme étant de la glucose oxydase ou de la cholestérol oxydase.

7. Méthode pour améliorer la stabilité d'une composition qui contient une enzyme et un composé ferricyanure comme médiateur soluble qui sera réduit en réponse à l'action de l'enzyme sur un analyte pour lequel il est spécifique, la méthode comportant l'addition, à la composition, d'environ 1,3x10⁻⁴ grammes par kU d'enzyme à environ 17,5x10⁻⁴ grammes par kU d'enzyme d'un dichromate de métal alcalin qui est soluble dans la composition de réactifs et qui ne réussira pas à entrer en concurrence avec la réduction du composé de ferricyanure en réponse à l'action de l'enzyme sur l'analyte qui peut être présent dans l'échantillon, mais qui inhibera la réduction prématurée du composé ferricyanure.

8. Méthode selon la revendication 7, le dichromate de métal alcalin étant choisi dans le groupe constitué par le dichromate de sodium et le dichromate de potassium.

9. Méthode selon la revendication 7, l'enzyme étant de la glucose oxydase ou de la cholestérol oxydase.

10. Méthode selon la revendication 7, l'enzyme étant de la glucose oxydase et le dichromate de métal alcalin étant du dichromate de potassium.

11. Méthode selon la revendication 7, la composition contenant en outre un composé ferrique soluble qui réagit avec un composé ferrocyanure, afin de former un produit de réaction visuellement ou optiquement détectable, et un tampon qui n'empêche pas la formation du produit de réaction, la composition présentant un pH compris entre environ 3,0 et environ 7,0.

12. Méthode selon la revendication 11, le dichromate de métal alcalin étant choisi dans le groupe constitué par le dichromate de sodium et le dichromate de potassium.

13. Méthode selon la revendication 11, l'enzyme étant de la glucose oxydase ou de la cholestérol oxydase.

14. Méthode selon la revendication 13, l'enzyme étant de la glucose oxydase et le dichromate de métal alcalin étant du dichromate de potassium.
